# EUROPEAN PATENT SPECIFICATION

(11) **EP 0 521 094 B1**
(45) Date of publication and mention of the grant of the patent: **14.09.1994**
(21) Application number: 91907218.1
(22) Date of filing: 18.03.1991
(51) Int. Cl.: A01N 63/00, A01N 63/02, C12N 1/20

(54) **USE OF A COLEOPTERAN ACTIVE $i(BACILLUS THURINGIENSIS) FOR CONTROLLING THE CEREAL LEAF BEETLE $i(OULEMA MELANOPA L. (LEMA MELANOPA (L.)))**
VERWENDUNG EINES GEGEN COLEOPTERA WIRKSAMEN BACILLUS THURINGIENSIS ZUR BEKÄMPFUNG DES GETREIDEBLATTHÄHNCHENS OULEMA MELANOPA (L.) (LEMA MELANOPA (L.))
UTILISATION DU $i(BACILLUS THURINGIENSIS) POUR LUTTER CONTRE LE COLEOPTERE DES FEUILLES DE CEREALES $i(OULEMA MELANOPA L. (LEMA MELANOPA (L.)))

(30) Priority: 22.03.1990 DK 738/90
(43) Date of publication of application: 07.01.1993
(73) Proprietor: NOVO NORDISK A/S, 2880 Bagsvaerd (DK)
(72) Inventor: RUGH, Susanne, DK-2880 Bagsvaerd (DK)
(74) Representative: Jensen, Bo Hammer
(86) International application number: DK9100081
(87) International publication number: WO9114369

(56) References cited:
- EP-A- 0 149 162
- EP-A- 0 382 990
- US-A- 4 766 203
- US-A- 4 797 276
- International Colloquium of Invertebrate Pathology, vol., 4, 1986 Robert A. Samson et al.: "Fundamental and applied aspects of invertebrate pathology", see page 555.
- Environmental entomology, vol., 19, no. 2, 1990 L. S. Bauer: "Response of the Cottonwood Leaf Beetle (Coleoptera: Chrysomelidae) to Bacillus thuringiensis var. san diego", see page 428-431.

## Description

### FIELD OF THE INVENTION

This invention relates to the use of a coleopteran active B. thuringiensis or its delta endotoxin in controlling the cereal leaf beetle Oulema melanopa (L.) (Lema melanopa (L.)), a method of controlling Oulema melanopa (L.) (Lema melanopa (L.)) by applying an effective amount of coleopteran active B. thuringiensis or its delta endotoxin to an area infested by Oulema melanopa (L.) (Lema melanopa (L.)), and insecticidal compositions for controlling Oulema melanopa (L.) (Lema melanopa (L.)).

### BACKGROUND OF THE INVENTION

Commercial preparations of Bacillus thuringiensis are used worldwide for biological control of pest insects. The advantages of these bacterial insecticides are that they are highly selective for a very limited range of target insects and are biodegradable.

Commercial preparations of Bacillus thuringiensis can be applied right up to the time of harvest with no adverse effects.

Bacillus thuringiensis is a rod shaped, aerobic, spore forming bacterium uniquely characterized by the production during the sporulation process of one or more inclusions, referred to as parasporal crystals. These crystals are composed of high molecular weight proteins, referred to as delta-endotoxins. The delta-endotoxins are the active ingredient in available commercial preparations of Bacillus thuringiensis.

Many B. thuringiensis strains with different insect host spectra have been identified. They are classified into different subspecies based on their flagellar antigens. Of particular interest is Bacillus thuringiensis subspecies kurstaki and subspecies aizawai used for the control of lepidopteran pest insects, Bacillus thuringiensis subspecies israelensis used for the control of dipteran pest insects and Bacillus thuringiensis subspecies tenebrionis and Bacillus thuringiensis subspecies San Diego used for the control of coleopteran pest insects.

The first isolation of a coleopteran toxic Bacillus thuringiensis was reported in 1983 (A. Krieg et al., Z. ang. Ent. 96, 500-508, European Patent Publication EP 0149162 A2).

The isolate, which was designated Bacillus thuringiensis subsp. tenebrionis, has been deposited with the German Collection of Microorganisms under accession number DSM 2803. Bacillus thuringiensis subsp. tenebrionis was isolated in 1982 from a dead pupa of the mealworm, Tenebrio molitor (Tenebrionidae, Coleoptera). The strain produces within each cell one spore and one insecticidal parasporal crystal which is of flat plate-like form with an edge length of about 0.8 µm to 1.5 µm. It belongs to serotype H8a,8b and pathotype C of Bacillus thuringiensis (Krieg et al., System.Appl.Microbiol. 9, 138-141, 1987, US patent 4,766,203, 1988).

It is only toxic against certain leaf eating larvae (Chrysomelidae), but ineffective against caterpillars (Lepidoptera), mosquitoes (Diptera) or other insects.

Coleopteran active Bacillus thuringiensis has been shown to be an effective control agent for the colorado potato beetle larvae. After uptake of crystals and spores from coleopteran active Bacillus thuringiensis or isolated crystals larvae, and to a certain extent adults, of the colorado potato beetle (Leptinotarsa decemlineata) stop feeding. Larvae stages L1-L3 die within 1-3 days (Schnetter et al., in "Fundamental & applied aspects of invertebrate pathology", eds. R.A. Samson et al., Proceedings of the 4th Int. colloquium of Invertebrate Pathology, p. 555, 1986).

The activity of B. thuringiensis subsp tenebrionis and San Diego has further been confirmed against the elm leaf beetle Xanthogaleruca luteola.

However, it has also been found that coleopteran active B. thuringiensis does not exhibit activity towards all insects within the Chrysomelidae. It has for example been found that it has no activity towards the small black flea beetle, Phyllotreta atra and the small striped flea beetle, Phyllotreta undulata, and only minor activity against the banded cucumber beetle, Diabrotica balteata, and the asparagus beetle, Crioseres asparagi.

Four commercial products of coleopteran active Bacillus thuringiensis have been developed for the control of coleopteran pests. NOVODOR® from Novo Biokontrol, Novo Nordisk A/S, TRIDENT® from Sandoz, and DiTerra® from Abbott Laboratories Inc. all based on Bacillus thuringiensis subspecies tenebrionis, and the commercial product M-one® from Mycogen Corporation based on a closely related strain Bacillus thuringiensis subsp. San Diego.

### SUMMARY OF THE INVENTION

It has now been found that coleopteran active B. thuringiensis delta endotoxin exhibits activity towards a cereal leaf beetle Oulema melanopa (L.) (Lema melanopa (L.)).

This invention consequently relates to the use of coleopteran active B. thuringiensis or its delta endotoxin in controlling the cereal leaf beetle Oulema melanopa (L.) (Lema melanopa (L.)). Generally this is done in grasses, and especially in cereal crops.

The present invention also relates to a method of controlling the cereal leaf beetle Oulema melanopa (L.) (Lema melanopa (L.)) by applying an effective amount of a coleopteran active B. thuringiensis or its delta endotoxin to an area infested by Oulema melanopa (L.) (Lema melanopa (L.)).

The invention further relates to insecticidal compositions for controlling the cereal leaf beetle Oulema melanopa (L.) (Lema melanopa (L.)).

### DETAILED DESCRIPTION OF THE INVENTION

As indicated above the present invention in its first aspect relates to the use of coleopteran active B. thuringiensis products such as, NOVODOR®, for controlling the cereal leaf beetle Oulema melanopa (L.) (Lema melanopa (L.)). This beetle attacks the leaves of plants belonging to the grasses (monocotyledons), and consequently such products may generally be applied to grasses.

Of special economic importance are cereal crops belonging to the grasses, such as wheat, barley, oat, rye, rice, etc., where the use of coleopteran active B. thuringiensis products is of special value.

The cereal leaf beetle Oulema melanopa (L.) (Lema melanopa (L.)) belongs to the family Chrysomelidae. It is known as a serious pest widely distributed on the Eurasian continent from Sweden in the north to a latitude about Morocco, and from France to China, North Africa, and the north easterly parts of the U.S.A..

In its second aspect the invention relates to a method of controlling the leaf beetle Oulema melanopa (L.) (Lema melanopa (L.)) by applying an effective amount of a coleopteran active B. thuringiensis product to an area infested with said cereal leaf beetle. In this context the expression "effective amount" should be understood as single or repeated applications of from 0.5 l/ha to 8 l/ha of flowable preparations or 0.25 kg/ha to 4kg/ha of powder or granular preparations.

The invention in its third aspect relates to pesticidal compositions or preparations comprising the coleopteran active B. thuringiensis delta endotoxin product in admixture with an agriculturally acceptable diluent or carrier for use in controlling Oulema melanopa (L.) (Lema melanopa (L.)).

The compositions of the invention can take any form known in the art for the formulation of agrochemicals, for example, a suspension, a dispersion, an aqueous emulsion, a dusting powder, a dispersible powder, an emulsifiable concentrate or granules. Moreover they can be in a suitable form for direct application or as a concentrate or primary composition which requires dilution with a suitable quantity of water or other diluent before application.

The concentration of the insecticidally active B. thuringiensis delta endotoxir in the compositions of the present invention when used alone or in combination with another pesticide, as applied to plants is preferably within the range from about 0.5 to about 25 per cent by weight, especially 1 to 15 per cent by weight.

The coleopteran active B. thuringiensis preparation or the compositions of the invention can be applied directly to the plant by, for example, spraying or dusting at the time when the pest has begun to appear on the plant. The preferred mode of application is by spraying. It is generally important to obtain good control of pests in the early stages of pest development as this most effectively prevents crop damage.

The invention is illustrated in the following example showing a specific embodiment of this invention. The example should in no way be construed as limiting for the scope of the invention as defined in the appended claims.

### Example 1

In a field trial the product NOVODOR® FC was evaluated for its efficacy against the L1 & L2 larval stages of the cereal leaf beetle Oulema melanopa (L.) (Lema melanopa (L.)).

In the trial the insecticide was applied to various cereal crops as indicated below:
The NOVODOR® FC product is an aqueous flowable concentrate formulated with microbial stabilizers, detergents and suspension aids.

| | | |
|---|---|---|
| Crop: | Spring Wheat | 2 varieties |
| | Spring Barley | 1 variety |
| | Winter Wheat | 2 varieties |
| | Tricale | 8 varieties |

For comparison 3 traditional pesticides DECIS® (from Roussel Uclaf), KARATE® (from ICI), and SUMIALFA® (from SUMITOMO) were also tested, all these pesticides are of the pyrethroid type well known in the art.

The pesticides were applied in a single application in the following doses:

| Product | Dose l/ha |
|---|---|
| NOVODOR® FC | 2.5 |
| NOVODOR® FC | 5.0 |
| DECIS® 2.5EC | 250 ml/ha |
| KARATE® 2.5EC | 250 ml/ha |
| SUMIALFA® 5EC | 200 ml/ha |

and at the 50% hatching time for the larvae.

The evaluation was performed according to the following procedure:
The numbers of larvae dead or alive at 1, 3, 5, 10 & 14 days post spray were counted, and the percentage of dead larvae computed as an average of 13 experiments for the NOVODOR® FC product, and as an average of 3 experiments for the traditional products.

The results are presented in Table I below:

**TABLE I**

| Product | Dose l/ha | % dead after days (av. of 13 reps) | | | | |
|---|---|---|---|---|---|---|
| | | 1 | 3 | 5 | 10 | 14 |
| NOVODOR® FC | 2.5 | 80 | 90 | 100 | 95 | 85 |
| NOVODOR® FC | 5.0 | 80 | 95 | 100 | 100 | 85 |
| Pyrethroids | av. of 3 | 100 | 100 | 100 | 100 | 100 |
| Observations: for NOVODOR® FC Day 1: Larvae alive but not motile Day 3: Dead larvae observed amongst the live & non motile forms Day 5: Larvae mostly dead, some non motile forms Day 10: Most leaves free of larvae; few L2 & L3 larvae seen Day 14: Few L3 & L4 larvae seen. Damage negligible. | | | | | | |

From the results it is apparent that NOVODOR® FC at 2.5 & 5.0 l/ha gave excellent control of Oulema melanopa (L.) (Lema melanopa (L.)) larvae, thereby reducing the foliar damage from their attack to negligible proportions.

## Claims

1. Use of at least one coleopteran active Bacillus thuringiensis or its delta endotoxin for controlling the cereal leaf beetle Oulema melanopa (L.) (Lema melanopa (L.)).

2. Use according to claim 1, wherein said coleopteran active Bacillus thuringiensis is Bacillus thuringiensis subspecies tenebrionis or Bacillus thuringiensis subsp. San Diego, preferably Bacillus thuringiensis subspecies tenebrionis.

3. Use according to any of claims 1 or 2 in grasses.

4. Use according to any of claims 1 to 3 in cereal crops.

5. A method for controlling the cereal leaf beetle Oulema melanopa (L.) (Lema melanopa (L.)), wherein an effective amount of a coleopteran active Bacillus thuringiensis product is applied to plants infested with Oulema melanopa (L.) (Lema melanopa (L.))

6. The method of claim 5, wherein said coleopteran active Bacillus thuringiensis product originates from Bacillus thuringiensis subspecies tenebrionis or Bacillus thuringiensis subsp. San Diego, preferably Bacillus thuringiensis subspecies tenebrionis.

7. The method of claim 6, wherein a liquid product is applied in an amount of 0.5 l/ha to 8 l/ha, preferably 0.5 l/ha to 4 l/ha.

8. The method of claim 6, wherein a flowable powder or granular product is applied in an amount of 0.25 kg/ha to 5 kg/ha, preferably 0.5 kg/ha to 2 kg/ha.

9. The method of claim 6, wherein a wetable powder product is applied in an amount of 0.25 kg/ha to 5 kg/ha, preferably 0.5 kg/ha to 2 kg/ha.

10. The method of claim 6, 7, 8, or 9, wherein the product is applied repeatedly with intervals.

## Patentansprüche

1. Verwendung von mindestens einem *Coleoptera*-aktiven Bacillus thuringiensis oder seines Delta-Endotoxins zum Kontrollieren des Getreideblattkäfers Oulema melanopa (L.) (Lema melanopa (L.)).

2. Verwendung nach Anspruch 1, wobei der Coleoptera-aktive Bacillus thuringiensis Bacillus thuringiensis subspecies tenebrionis oder Bacillus thuringiensis subsp. San Diego, vorzugsweise Bacillus thuringiensis subspecies tenebrionis, ist.

3. Verwendung nach einem der Ansprüche 1 oder 2 bei Gräsern.

4. Verwendung nach einem der Ansprüche 1 bis 3 bei Getreidearten.

5. Verfahren zum Kontrollieren des Getreideblattkäfers Oulema melanopa (L.) (Lema melanopa (L.)), wobei eine wirksame Menge eines *Coleoptera*-aktiven Bacillus thuringiensis-Produktes bei Pflanzen angewendet wird, die von Oulema melanopa (L.) (Lema melanopa (L.)) befallen sind.

6. Verfahren nach Anspruch 5, wobei das *Coleoptera*-aktive Bacillus thuringiensis-Produkt von Bacillus thuringiensis subspecies tenebrionis oder Bacillus thuringiensis subsp. San Diego, vorzugsweise Bacillus thuringiensis subspecies tenebrionis, abstammt.

7. Verfahren nach Anspruch 6, wobei ein flüssiges Produkt in einer Menge von 0,5 l/ha bis 8 l/ha, vorzugsweise 0,5 l/ha bis 4 l/ha, angewendet wird.

8. Verfahren nach Anspruch 6, wobei ein fließfähiger Puder oder ein Granulat in einer Menge von 0,25 kg/ha bis 5 kg/ha, vorzugsweise 0,5 kg/ha bis 2 kg/ha, angewendet wird.

9. Verfahren nach Anspruch 6, wobei ein benetzbares Puderprodukt in einer Menge von 0,25 kg/ha bis 5 kg/ha, vorzugsweise 0,5 kg/ha bis 2 kg/ha, angewendet wird.

10. Verfahren nach Anspruch 6, 7, 8 oder 9, wobei das Produkt wiederholt in Intervallen angewendet wird.

## Revendications

1. L' utilisation d' au moins un Bacillus thuringiensis *coléoptère*-actif ou sa delta-endotoxine pour contrôler les coléoptères Oulema melanopa (L.) (Lema melanopa (L.)).

2. L' utilisation selon la revendication 1, pour laquelle le Bacillus thuringiensis coléoptère-actif est Bacillus thuringiensis subspecies tenebrionis ou Bacillus thuringiensis subsp. San Diego, de préférence Bacillus thuringiensis subspecies tenebrionis.

3. L' utilisation selon une des revendications 1 ou 2 pour des graminées.

4. L' utilisation selon une des revendications 1 à 3 pour des espèces de céréales.

5. Un procédé pour contrôler les coléoptères Oulema melanopa (L.) (Lema melanopa (L.)), dans lequel on applique une quantité efficace d'un produit Bacillus thuringiensis *coléoptère*-actif à des plantes attaquée par Oulema melanopa (L.) (Lema melanopa (L)).

6. Un procédé selon la revendication 5, dans lequel le produit Bacillus thuringiensis *coléoptère*-actif est dérivé d'un Bacillus thuringiensis subspecies tenebrionis ou de Bacillus thuringiensis subsp. San Diego, de préférence de Bacillus thuringiensis subspecies tenebrionis.

7. Un procédé selon la revendication 6, dans lequel on applique une quantité d'un produit liquide de 0,5 l/ha à 8 l/ha, de préférence 0,5 l/ha à 4 l/ha.

8. Un procédé selon la revendication 6, dans lequel on applique une quantité d'un poudre coulant ou d'un produit granulaire de 0,25 kg/ha à 5 kg/ha, de préférence de 0,5 kg/ha à 2 kg/ha.

9. Un procédé selon la revendication 6, dans lequel on applique un produit d'un poudre mouillable de 0,25 kg/ha à 5 kg/ha, de préférence de 0,25 kg/ha à 2 kg/ha.

10. Un procédé selon la revendication 6, 7, 8 ou 9, dans lequel on applique le produit à plusieurs reprises en intervalles.
